# EUROPEAN PATENT APPLICATION

(11) **EP 4 302 797 A1**
(43) Date of publication of application: **10.01.2024**
(21) Application number: 22775106.2
(22) Date of filing: 09.03.2022
(51) Int. Cl.: A61M 5/14, A61M 5/168, A61M 25/00, A61M 25/06

(54) **MEDICAL INSTRUMENT**

(30) Priority: 23.03.2021 JP 2021048594
(71) Applicant: TERUMO Kabushiki Kaisha, Tokyo 151-0072 (JP)
(72) Inventor: ARIMA, Hiroki, Ashigarakami-gun, Kanagawa 259-0151 (JP); KOMEDA, Yoshiki, Ashigarakami-gun, Kanagawa 259-0151 (JP)
(74) Representative: Casalonga
(86) International application number: PCT/JP2022/010221
(87) International publication number: WO 2022/202314

(57) **Abstract**

A medical device 100 includes an injection unit 140 connectable to a barrel 110 that is capable of containing a drug and having internal spaces 152 and 162 that allow passage of the drug and includes a first measurement unit 170 that measures a pressure at a first part P1 in the distal end side of the internal spaces and a pressure at a second part P2 closer to the proximal end side of the internal spaces than the first part.

## Description

### Technical Field

The present invention relates to a medical device.

### Background Art

To treat a tumor developed in the liver, percutaneous ethanol injection (for example, see Patent Literature 1) may be employed for local treatment to cause necrosis of a cancer cell. An operator incises the abdomen or chest, punctures the liver with a needle cannula, and brings the needle tip to an affected area of liver cancer. The operator injects ethanol from the needle tip to induce necrosis of the cancer cell.

### Citation List

### Patent Literature

Patent Literature 1: JP 4588977 B1

### Summary of Invention

### Technical Problem

The inventors have intensively studied how to check (understand) precisely that a drug is administered to an affected area in a tumor or the like.

The invention has been made in light of the problem, and an object of the invention is to provide a medical device capable of obtaining information on how a drug is administered to an affected area in a tumor or the like.

### Solution to Problem

To achieve the object, a medical device according to an aspect of the invention includes an injection unit and a first measurement unit. The injection unit includes a channel connectable to a drug container that is capable of containing a drug and configured to allow passage of the drug. The first measurement unit obtains a pressure difference between a first part in the distal end side of the channel and a second part closer to the proximal end side of the channel than the first part.

### Advantageous Effects of Invention

According to the medical device, it is possible to obtain information on how a drug is administered to an affected area in a tumor or the like.

### Brief Description of Drawings

Fig. 1 is a schematic perspective view of a medical device according to a first embodiment of the invention.
Fig. 2 is a view illustrating the inside of an injection unit of the medical device illustrated in Fig. 1 and other components of the medical device.
Fig. 3 is a view illustrating the inside of a needle member according to a modification.
Fig. 4 is a cross-sectional view intersecting with a longitudinal direction of the needle member illustrated in Fig. 3.
Fig. 5 is a view illustrating the inside of a needle member included in an injection unit of a medical device according to a modification provided with a second measurement unit.
Fig. 6 is a cross-sectional view intersecting with the longitudinal direction of the needle member illustrated in Fig. 5.
Fig. 7 is a view of a connecting member according to a modification, illustrating the inside of an injection unit and other components of a medical device.
Fig. 8 is a view of a connecting member according to a modification, illustrating the inside of an injection unit and other components of a medical device.

### Description of Embodiments

Hereinafter, modes for carrying out the invention will be described in detail with reference to the drawings. Embodiments herein are illustrated to embody the technical idea of the invention and do not limit the invention. Other embodiments, examples, technical operations, and the like that could be conceived by those skilled in the art without departing from the gist of the invention are all included in the scope and gist of the invention and included in the invention disclosed in the claims and the scope of equivalents thereof.

Furthermore, for the purpose of illustration and for ease of comprehension, the scale, aspect ratio, shape, and the like in the drawings attached may be changed from actual ones as appropriate and shown schematically. However, it is noteworthy that the drawings are examples and do not limit the interpretation of the invention.

Moreover, in the following description, the ordinal numerals such as "first" and "second" are given, but they are used for convenience sake and do not specify any sequential order unless otherwise specified.

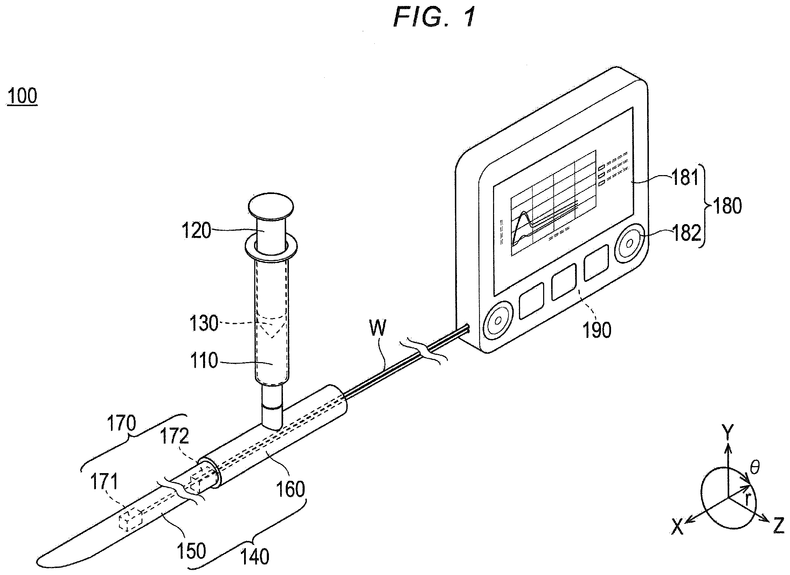

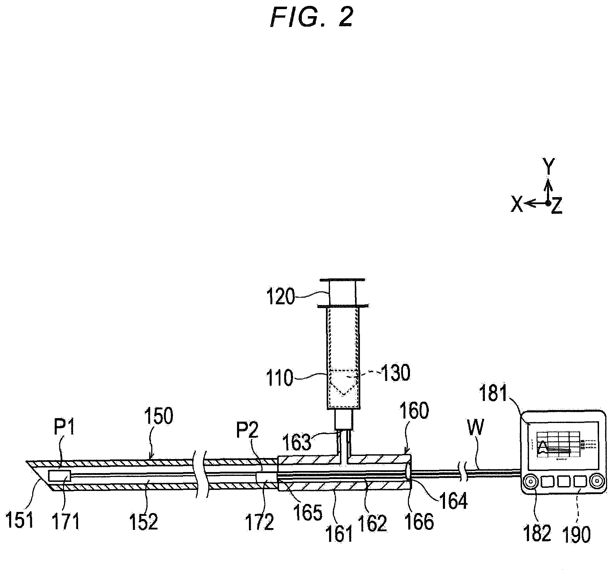

Hereinafter, a medical device 100 according to a first embodiment will be described with reference to Figs. 1 and 2. Fig. 1 is a schematic perspective view of the medical device 100 according to the first embodiment. Fig. 2 is a view illustrating the inside of an injection unit 140 included in the medical device 100 illustrated in Fig. 1 and other components of the medical device 100.

Each drawing shows a coordinate system. X represents a longitudinal direction of a needle member 150 or 150a (hereinafter referred to as "needle member 150 or the like") included in the injection unit 140 or 140a and is referred to as "longitudinal direction X." Y and Z represent a plane intersecting with (perpendicular to) the longitudinal direction X and are referred to as "YZ-plane". The alphabet r represents a radial direction from the center of the needle member 150 or the like in YZ-plane and is referred to as "radial direction r." The symbol 0 represents a circumferential direction along the circumference (angular direction) of the needle member 150 or the like in YZ-plane intersecting with the longitudinal direction X of the needle member 150 or the like and is referred to as "circumferential direction θ."

The medical device 100 according to this embodiment is used for administration of an anticancer drug or other drugs to a patient.

To describe briefly with reference to Figs. 1 and 2, the medical device 100 according to this embodiment includes a barrel 110 (corresponding to a drug container), a pressing member 120, a sealing member 130, the injection unit 140, a first measurement unit 170, a notification unit 180, and a control unit 190. Each configuration will now be described in detail.

### (Barrel)

The barrel 110 is provided with a semi-closed space that contains a drug such as anticancer drugs. The barrel 110 has a tubular shape like a cylinder, having openings at both ends of the tubular shape in an axial direction. In one opening (also referred to as "proximal end side opening"), the pressing member 120 is movably disposed. To the other opening (also referred to as "distal end side opening"), a connecting member 160 included in the injection unit 140 is attached.

### (Pressing Member)

The pressing member 120 has the distal end side housed in the semi-closed space of the barrel 110 and the proximal end side disposed outside the barrel 110. The pressing member 120 moves relative to the barrel 110 in the axial direction (Y direction intersecting with the longitudinal direction X) of the barrel 110 so as to change the size of the semi-closed space containing the drug. Shrinking the semi-closed space by the pressing member 120 decreases an amount of drug contained in the semi-closed space, and the equivalent amount of drug flows through internal spaces 152 and 162 in the injection unit 140 and is administered to a patient.

### (Sealing Member)

The sealing member 130 is attached to a distal end of the pressing member 120 in the axial direction (Y direction intersecting with the longitudinal direction X). The sealing member 130 is slidably fitted into an inner wall of the barrel 110,
being configured to prevent the drug contained in the semi-closed space in the barrel 110 from flowing through places other than the internal spaces 152 and 162 included in the injection unit 140.

### (Injection Unit)

The injection unit 140 is connectable to the distal end side opening of the barrel 110 containing the drug. The injection unit 140 includes the needle member 150 and the connecting member 160 as illustrated in Fig. 1.

The needle member 150 is insertable into the body of a patient. The needle member 150 includes an outlet 151 and the internal space 152. The needle member 150 has a hollow elongated tubular shape, and the outlet 151 includes an opening for releasing the drug passing through the internal space 152 to the outside. The outlet 151 is opened at a distal end in the longitudinal direction X in this embodiment. The opening of the outlet does not necessarily face the distal end in the longitudinal direction X as long as the drug is released to a tumor while the injection unit 140 is placed in a body lumen.

Note that the needle member 150 having the tubular shape is illustrated as a cylinder in this embodiment but may be a cuboid.

The internal space 152 constitutes an elongated channel that allows passage of the drug from the barrel 110. The internal space 152 supplies the drug from the barrel 110 to the outlet 151. The internal space 152 is continuous with the semi-closed space of the barrel 110. The needle member 150 in this embodiment has a uniform width in the radial direction r intersecting with the longitudinal direction X. The needle member 150 that forms the internal space 152 does not necessarily have a uniform width in the radial direction r as long as the drug passes through the internal space 152.

The needle member 150 is not particularly limited in size and may have, for example, a length of 300 mm in the longitudinal direction X and an inside diameter of 0.3 to 0.5 mm.

The connecting member 160 includes, as illustrated in Fig. 2, a main body 161, the internal space 162, a first opening 163, a second opening 164, a third opening 165, and a valve 166.

The main body 161 is connected to the distal end side of the barrel 110 and also to the proximal end side of the needle member 150, being configured to guide wires W from a first sensor 171 and a second sensor 172 included in the first measurement unit 170 to the outside.

Together with the internal space 152 of the needle member 150, the internal space 162 constitutes the elongated channel that allows passage of the drug. The internal space 162 is equivalent or similar to the needle member 150 in size that defines a cross-sectional shape such as a diameter in a cross section intersecting with the longitudinal direction X of a part connecting the second opening 164 and the third opening 165.

The first opening 163 in this embodiment is connected to the distal end side of the barrel 110, facing Y direction intersecting with the longitudinal direction X. The first opening 163 connected to the barrel 110 and the second opening 164 for guiding the wires W from the first measurement unit 170 are disposed in different parts.

The second opening 164 guides the wires W of the first sensor 171 and the second sensor 172 included in the first measurement unit 170 to the outside of the internal space 162. The second opening 164 in this embodiment faces the longitudinal direction X and is placed opposite to the third opening 165. The second opening 164 is provided with the valve 166 so as to prevent passage of the drug from the second opening 164 to the outside of the internal space 162.

The third opening 165 is a part connected to the proximal end side of the needle member 150 included in the injection unit 140. The third opening 165 in this embodiment faces the longitudinal direction X.

The barrel 110, pressing member 120, sealing member 130, needle member 150, and the main body 161 and valve 166 of the connecting member 160 included in the medical device 100 are not particularly limited in material as long as the drug is administered to a patient through the needle member 150. Examples of the material in the barrel 110, pressing member 120, and main body 161 include plastic materials such as polypropylene and polyethylene, and examples of the material in the sealing member 130 and valve 166 include butyl rubber, silicone rubber, and elastomer. An example of the material in the needle member 150 includes stainless steel.

### (First Measurement Unit)

The first measurement unit 170 measures pressures at a first part P1 in the distal end side of the internal spaces 152 and 162 of the injection unit 140 and at a second part P2 closer to the proximal end side than the first part P1. The first measurement unit 170 includes, as illustrated in Fig. 2, the first sensor 171 and the second sensor 172.

As described above, the internal space of the injection unit 140 in this embodiment includes the internal space 152 of the needle member 150 and the internal space 162 of the connecting member 160. As illustrated in Fig. 2, the first sensor 171 is disposed near the outlet 151 in the distal end side of the needle member 150. Herein, this point is referred to as the first part P1.

The second sensor 172 is disposed closer to the proximal end side than the first sensor 171 in the internal spaces 152 and 162. The second sensor 172 in this embodiment is disposed in the proximal end side of the internal space 152 of the needle member 150. Herein, this point is referred to as the second part P2. The first sensor 171 and the second sensor 172 in this embodiment are fixed to the internal space 152 of the needle member 150 or the like with an adhesive or by thermal fusion or stapling.

Sensors of the first sensor 171 and the second sensor 172 are not particularly limited as long as they measure a pressure difference in the internal space of the injection unit. The first sensor 171 and the second sensor 172 are, for example, diaphragm sensors capable of measuring pressures at the first part P1 and the second part P2, respectively, and have a cross section intersecting with the longitudinal direction X formed into a 0.1 mm square.

The first sensor 171 and the second sensor 172 included in the first measurement unit 170 are configured to measure pressures while being connected to the wires W.

### (Notification Unit)

The notification unit 180 notifies an operator or other users of information associated with the pressures measured by the first measurement unit 170. The notification unit 180 includes, as illustrated in Fig. 1 and the like, a visual alert unit 181 and an auditory alert unit 182.

The visual alert unit 181 is electrically connected to the control unit 190 and visually displays the results measured by the first measurement unit 170. The visual alert unit 181 may include a known display such as liquid crystal display and OLED. The visual alert unit 181 displays the pressures measured by the first measurement unit 170 using numerical values and graphs.

The auditory alert unit 182 operates according to a pressure difference between two points in the internal space 152 and 162 of the injection unit 140 measured by the first measurement unit 170. The auditory alert unit 1 82 notifies a user that values measured by the first measurement unit 170 satisfy a specific condition. The specific condition in this embodiment refers to a case where the pressures measured by the first sensor 171 and the second sensor 172 exceed a threshold.

In a case where a pressure difference between two points in internal spaces 152 and 162 is below a predetermined threshold, it is likely that the drug is released to a closed space like a tumor developed in a patient. In a case where a pressure difference is over the predetermined threshold, it is likely that the drug is released to a ruptured tumor or to an open space as in a case where the drug is delivered through a blood vessel.

Based on this relation, the auditory alert unit 182 emits sounds to notify the operator or other users when a pressure difference between two points in the internal spaces 152 and 162 exceeds the predetermined threshold. The predetermined threshold in this embodiment is designed to determine whether that the pressures measured by the first sensor 171 and the second sensor 172 are equal, that is, whether there is a pressure difference. The auditory alert unit 182 includes, for example, a speaker or other devices that emit sounds.

### (Control Unit)

The control unit 190 integrally controls the first measurement unit 170, the notification unit 180, and the like. The control unit includes a processor such as CPU and GPU and a storage unit, for example, a primary memory such as RAM and a secondary memory such as ROM, HDD, and SSD. The control unit 190 is housed in a casing including the notification unit 180 and the like illustrated in Fig. 1.

### (Usage Example)

Hereinafter described is a usage example of the medical device 100 according to this embodiment.

First, an operator moves the pressing member 120 relative to the barrel 110 and transfers the drug contained in the semi-closed space in the barrel 110 to the internal spaces 152 and 162 of the injection unit 140 provided with the first measurement unit 170 (priming).

In leaking the drug out by the priming, the operator should carefully move the pressing member 120 relative to the barrel 110 and put a tray or the like under the medical device 100 to prevent the drug from scattering to unintended places or unintended parts.

Next, the operator turns on a power supply (not illustrated) and causes the control unit 190 to be electrically connected to the first measurement unit 170 and the notification unit 180. The operator resets values of the first sensor 171 and the second sensor 172. Next, the operator makes a small incision around the abdomen of a patient. The operator inserts the needle member 150 of the injection unit 140 through the body surface to perform percutaneous puncture by ultrasonic echo and advances the distal end of the needle member 150 to the front of a tumor or into the tumor.

Next, the operator moves the pressing member 120 relative to the barrel 110 to reduce the semi-closed space in the barrel 110, thereby administering the drug to the patient. When pressures at two points in the internal spaces 152 and 162 of the injection unit 140 are measured while the needle member 150 is inserted into a closed space or open space, the inventors have found that measured values may vary due to pressure loss depending on the site punctured with the needle member 150.

When pressures at the two points are measured while the distal end of the needle member 150 is inserted into a closed space, measured values are equal. In contrast, when pressures at the two points are measured while the distal end of the needle member 150 is inserted into an open space, measured values are different, that is, the measured value obtained in the downstream section of the channel is lower than the measured value obtained in the upstream section.

The notification unit 180 causes the visual alert unit 181 to notify the operator of the results measured by the first measurement unit 170 in a visual way, for example, using numerical values. In a case where a pressure loss is found based on the results measured by the first measurement unit 170, the auditory alert unit 182 notifies the operator accordingly by sounds. In this manner, the medical device 100 according to this embodiment notifies an operator of information on how a drug is administered to an affected area in a tumor or the like.

After checking that the drug is administered to the tumor of the patient, the operator removes the medical device 100 from the body.

As described above, the medical device 100 according to this embodiment includes the injection unit 140 and the first measurement unit 170. The injection unit 140 is connectable to the barrel 110 that is capable of containing the drug and includes the internal spaces 152 and 162 that allow passage of the drug. The first measurement unit 170 measures pressures at the first part P1 in the distal end side of the internal spaces 152 and 162 and at the second part P2 closer to the proximal end side of the internal spaces 152 and 162 than the first part P1.

This configuration makes it possible to notify an operator or other users of information on how a drug is administered to an affected area, or an open space and closed space, of a patient.

The first measurement unit 170 is configured to measure pressures while being connected to the wires W. The injection unit 140 includes the connecting member 160 and the needle member 150 provided with the internal space 152 and insertable into the patient's body. The connecting member 160 is provided with the internal space 162 and connected to the needle member 150 in the proximal end side of the needle member 150, having openings different from each other, that is, the first opening 163 connected to the barrel 110 and the second opening 164 for guiding the wires W from the first measurement unit 170.

This configuration also makes it possible to notify an operator or other users of information on how a drug is administered to an affected area, or an open space and closed space, of a patient.

In addition, the first measurement unit 170 includes the first sensor 171 disposed in the first part P1 in the internal spaces 152 and 162 and the second sensor 172 disposed in the second part P2 in the internal space 152 and 162. This configuration enables measurement of pressures at two different points in the internal spaces 152 and 162 of the injection unit 140.

Furthermore, the first measurement unit 170 is fixed to the internal space 152. Accordingly, it is possible to prevent the sensors for measuring pressures at the first part P1 and the second part P2 from being changed in position by a flow of the drug, which reduces variation of measured values.

The medical device 100 also includes the visual alert unit 181 that visually displays the measured values representing the pressures measured by the first measurement unit 170. Accordingly, it is easier for a user to understand the results measured by the first measurement unit 170.

Moreover, the medical device 100 includes the notification unit 180 that emits sounds to notify a user that a difference of values at two points measured by the first measurement unit 170 satisfies a specific condition, that is, a difference of values exceeds a threshold. With this configuration, it is possible to notify an operator and other users in a tangible way when, for example, the drug is not administered as intended by the operator.

### (First Modification)

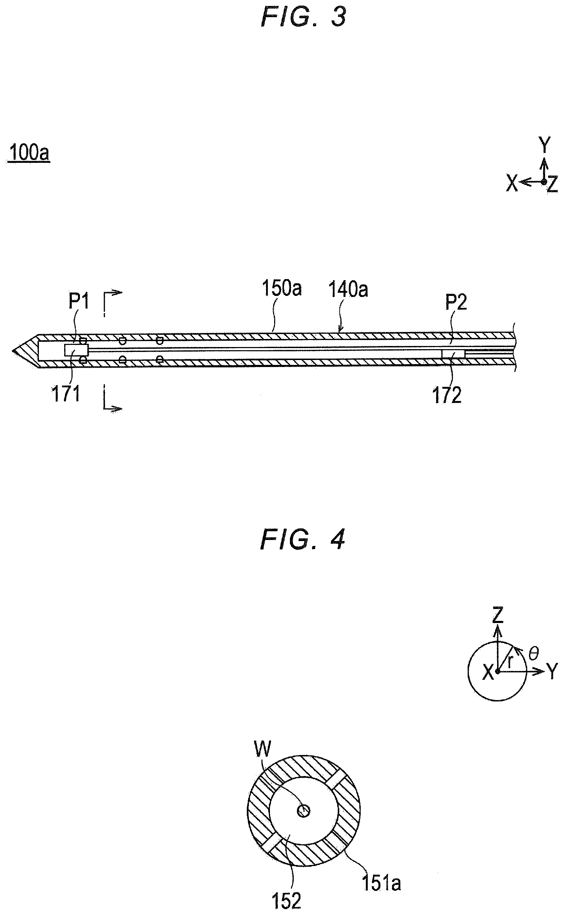

Fig. 3 is a cross-sectional view along the longitudinal direction X of a medical device 100a according to a first modification, illustrating the inside of a needle member 150a included in an injection unit 140a. Fig. 4 is a cross-sectional view intersecting with the longitudinal direction X of an outlet 151a of the needle member 150a.

In the first embodiment, the outlet 151 of the needle member 150 included in the injection unit 140 faces the distal end side of the needle member 150 in the longitudinal direction X. Note that the outlet does not necessarily face the distal end side in the longitudinal direction X as long as a drug is released to an affected area in a tumor or the like.

As illustrated in Figs. 3 and 4, the needle member 150a in this modification is provided with an internal space 152, having a tubular shape like a cylinder with a closed distal end, and including the outlet 151a disposed on the lateral surface of the tubular shape. The outlet 151a in this modification faces the radial direction r intersecting with the longitudinal direction X. In this modification, four outlets 151a are arranged at regular intervals in the circumferential direction θ as illustrated in Fig. 4. As long as the drug is released to an affected area, the number of outlets is not limited to four and the outlets are not necessarily arranged at regular intervals.

Although the medical device 100a according to this modification is different from the first embodiment in position and orientation of the outlets, other configurations are similar, and the usage of this medical device will be omitted.

As described above, the outlets 151a of the needle member 150a in this modification are disposed on the lateral surface of the tubular shape with the closed distal end. With this configuration, it is possible to encompass and protect a first sensor 171 and the like disposed in the distal end side of the internal space 152. Arranging the outlets 151a on the lateral surface of the tubular shape makes it possible to set the number and size of the outlets 151a appropriately regardless of the diameter of the tubular shape, which enhances the efficiency of drug release. Furthermore, providing the lateral surface of the tubular shape with the outlets 151a reduces injection resistance.

### (Second Modification)

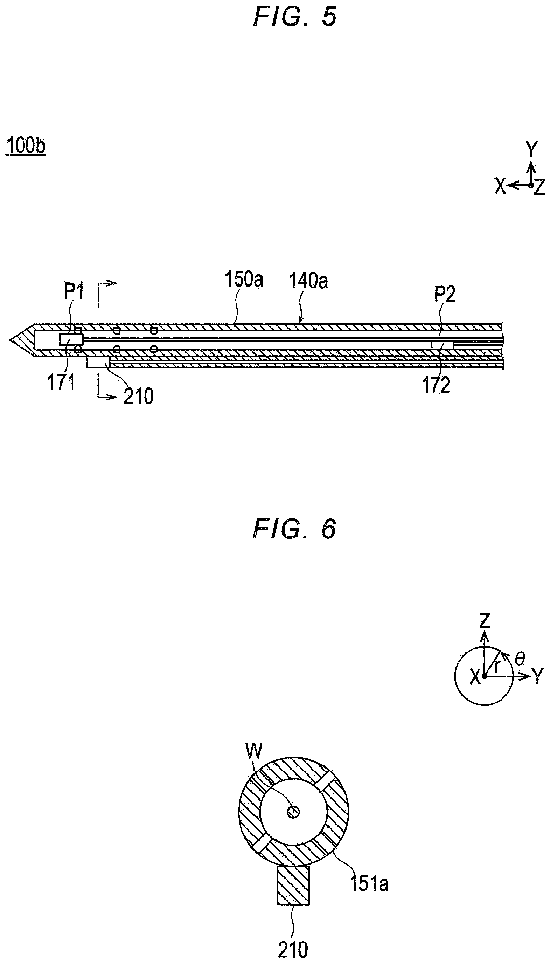

Fig. 5 is a cross-sectional view along the longitudinal direction X of a medical device 100b according to a second modification, illustrating the inside of a needle member 150a included in an injection unit 140a, a second measurement unit 210, and the like. Fig. 6 is a cross-sectional view intersecting with the longitudinal direction X of an outlet 151a of the needle member 150a, the second measurement unit 210, and the like. In the first embodiment, the first measurement unit 170 includes the first sensor 171 and the second sensor 172 disposed in the internal space 152 of the injection unit 140. In addition to the first sensor 171 and the second sensor 172, the following sensor may be added as a component for detecting a pressure loss.

The medical device 100b in this modification includes a barrel 110, a pressing member 120, a sealing member 130, the injection unit 140a, a first measurement unit 170, and the second measurement unit 210. Since the barrel 110, pressing member 120, sealing member 130, and first measurement unit 170 are similar to those in the first embodiment and the injection unit 140a is similar to that in the first modification, details will be omitted.

The second measurement unit 210 is disposed outside the needle member 150a included in the injection unit 140a, being configured to measure a pressure of a fluid existing around the injection unit 140a while the injection unit 140a is in contact with the fluid. The second measurement unit 210 may employ a sensor similar to the first sensor 171 and the second sensor 172 of the first measurement unit 170. The second measurement unit 210 is bonded to the outer surface of the needle member 150a of the injection unit 140a by thermal fusion or the like so as to be held on the surface in an integrated manner.

Hereinafter described is a usage example of the medical device 100b according to this modification. Since the priming is performed similarly to the first embodiment, details will be omitted.

Next, an operator turns on a power supply (not illustrated) and causes a control unit 190 to be electrically connected to the first measurement unit 170, a notification unit 180, and the second measurement unit 210. Next, the operator makes a small incision around the abdomen. The operator inserts the needle member 150a of the injection unit 140a through the body surface
to perform percutaneous puncture by ultrasonic echo and advances the distal end of the needle member 150a to the front of a tumor or into the tumor.

Next, similarly to the first embodiment, the operator moves the pressing member 120 relative to the barrel 110 to administer a drug to a patient. On the administration of the drug, in a case where the pressure measured by the first sensor 171 rises and the pressure measured by the sensor in the second measurement unit 210 does not rise, it is determined that internal spaces 152 and 162 of the injection unit 140a are clogged.

The operator can check the pressures measured by the first measurement unit 170 using the notification unit 180. Other configurations are similar to those in the first embodiment, and details will be omitted.

As described above, the medical device 100b according to this modification includes the second measurement unit 210 disposed outside the injection unit 140a and configured to measure a pressure of a fluid around the injection unit 140a while the injection unit 140a is in contact with the fluid.

Accordingly, it is possible to consider both values measured by the second measurement unit 210 and by the first measurement unit 170, which enables a user to be notified of information associated with the effect of drug administration by the injection unit 140a, for example, whether the internal spaces 152 and 162 of the injection unit 140a are clogged. In addition, even when the needle member 150a is clogged, it is possible to measure the intratumor pressure, and it is also possible to detect a drug leak.

### (Third Modification)

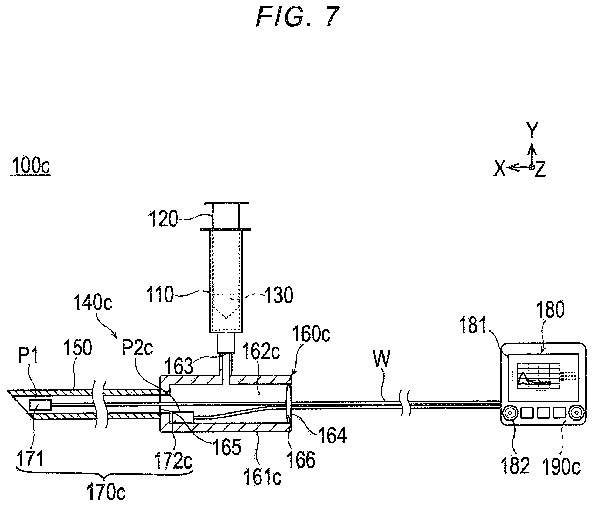

Fig. 7 is a view illustrating the inside of an injection unit 140c included in a medical device 100c according to a third modification and other components of the medical device 100c. In the first embodiment, with regard to the part connecting the second opening 164 and the third opening 165 of the connecting member 160, the cross section intersecting with the longitudinal direction X has a diameter similar to that of the needle member 150. However, the injection unit may have the following configuration.

The medical device 100c includes, as illustrated in Fig. 7, a barrel 110, a pressing member 120, a sealing member 130, the injection unit 140c, a first measurement unit 170c, a notification unit 180, and a control unit 190c. Since the barrel 110, pressing member 120, sealing member 130, and a needle member 150 included in the injection unit 140c are similar to those in the first embodiment, details will be omitted.

A connecting member 160c includes, as illustrated in Fig. 7, a main body 161c, an internal space 162c, a first opening 163, a second opening 164, a third opening 165, and a valve 166.

The main body 161c is connected to the barrel 110 and also to the needle member 150, being configured to guide wires W of a first sensor 171 and a second sensor 172 included in the first measurement unit 170c to the outside of the internal space 162c.

The internal space 162c is formed inside the main body 161c, being continuous with a semi-closed space of the barrel 110 that allows passage of a drug and continuous with an internal space 152 of the needle member 150 included in the injection unit 140c.

The internal space 162c allows passage of the drug and is provided with a second sensor 172c of the first measurement unit 170c. With regard to a diameter of a cross section intersecting with the longitudinal direction X of a part connecting the second opening 164 and the third opening 165, the internal space 162c where a second part P2c provided with the second sensor 172c is placed is larger than a first part P1 provided with the first sensor 171.

Since the first opening 163, second opening 164, third opening 165, and valve 166 are similar to those in the first embodiment, details will be omitted. The position of the second part P2c where the second sensor 172c is disposed is different from that in the first embodiment but other configurations are similar, and details of the first measurement unit 170c will be omitted.

The control unit 190c causes a storage unit to store a program for correcting measured pressures which vary due to a difference of the cross sections in the longitudinal direction X between the first part P1 and the second part P2c to assumed values which are to be obtained when the first part P1 and the second part P2c have an identical cross section. Other configurations of the control unit 190c are similar to those in the first embodiment, and details will be omitted.

The medical device 100c according to this modification is different from the first embodiment in that the medical device 100c corrects the variation of measured values due to a difference of the cross sections between the first part P1 and the second part P2c when measuring pressures at the time of drug injection, thereby determining whether a pressure loss is generated. However, other operations are similar to the first embodiment. Therefore, details of the usage will be omitted.

As described above, in this modification, the internal spaces 152 and 162c of the injection unit 140c are elongated, and the cross section intersecting with the longitudinal direction X of the second part P2c provided with the second sensor 172 is larger than that of the first part P1 provided with the first sensor 171. With this configuration, for example, when a needle tube of an insertion needle is bent, it is possible to reduce the effect caused by surrounding structures, that is, the effect caused by the fluctuation in pressure inside the needle tube of the needle member 150.

### (Fourth Modification)

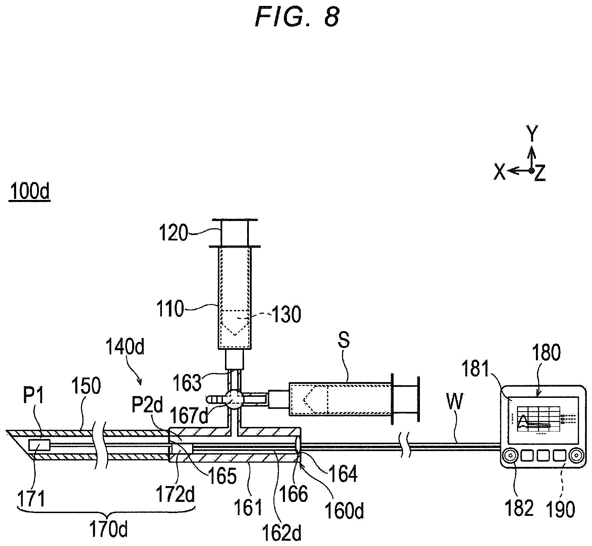

Fig. 8 is a view illustrating the inside of an injection unit 140d included in a medical device 100d according to a fourth modification and other components of the medical device 100d. A connecting member 160d included in the injection unit 140d of the medical device 100d may have the following configuration. Note that a barrel 110, a pressing member 120, a sealing member 130, and a needle member 150 included in the injection unit 140d are similar to those in the first embodiment, and details will be omitted.

The connecting member 160d includes, as illustrated in Fig. 8, a main body 161, an internal space 162d, a first opening 163, a second opening 164, a third opening 165, a valve 166, and a switching unit 167d. Since the main body 161, first opening 163, second opening 164, third opening 165, and valve 166 are similar to those in the first embodiment, details will be omitted.

The switching unit 167d includes a three-way stopcock capable of switching between connection with a syringe S or the like that makes the pressure of an internal space 152 and 162d negative and disconnection from the syringe S before drug administration. Although the internal space 162d has a different shape from the internal space 162 due to the switching unit 167d, a cross section intersecting with the longitudinal direction X of a part connecting the second opening 164 and the third opening 165 is similar to that of the needle member 150 and other parts are similar to the internal space 162.

A first measurement unit 170d includes a first sensor 171 and a second sensor 172d. Similarly to the first embodiment, the first sensor 171 is disposed in a first part P1 in the distal end side of the internal space 152. The second sensor 172d is disposed in a second part P2d, or the internal space 162d, of the connecting member 160d.

Hereinafter described is a usage example of the medical device 100d according to the fourth modification. First, an operator uses the three-way stopcock of the switching unit 167d to make a semi-closed space in the barrel 110 continuous with the internal spaces 152 and 162d included in the injection unit 140d and moves the pressing member 120 relative to the barrel 110, thereby performing priming as similar to the operation in the first embodiment.

Next, the operator turns on a power supply (not illustrated) and causes a control unit 190 to be electrically connected to the first measurement unit 170d and a notification unit 180. Next, the operator makes a small incision around the abdomen of a patient. The operator inserts the needle member 150 included in the injection unit 140d through the body surface to perform percutaneous puncture by ultrasonic echo and advances the distal end of the needle member 150 included in the injection unit 140d to the front of a tumor or into the tumor.

Next, similarly to the first embodiment, the operator moves the pressing member 120 relative to the barrel 110 to administer a drug to the patient. The operator obtains information associated with pressures at the first part P1 and the second part P2d measured by the first measurement unit 170d from a visual alert unit 181 or the like of the notification unit 180.

Next, the operator removes the medical device 100d from the body while the semi-closed space in the barrel 110 and the internal spaces 152 and 162d of the injection unit 140d are communicated by the switching unit 167d.

As described above, the medical device 100d in this modification includes the switching unit 167d such as a three-way stopcock that enables switching between connection of the connecting member 160d included in the injection unit 140d to the syringe S that makes the pressure of the internal space 152 and 162d negative and disconnection of the connecting member 160d from the syringe S.

Accordingly, it is possible to release pressures in the internal spaces 152 and 162d after the drug administration and it is possible to prevent a fluid leak when the needle member 150 is removed from the living body. Before the drug administration, the operator uses the switching unit 167d and attempts to make the internal spaces 152 and 162d negative by the syringe S or by the barrel 110 and the pressing member 120. When the pressures in the internal spaces 152 and 162d become negative, it is determined that the distal end of the needle member 150 is placed in a closed space such as a tumor and that a blood vessel is not punctured.

In contrast, when the pressures in the internal spaces 152 and 162d do not change to negative, it is determined that a blood vessel is punctured with the distal end of the needle member 150. In this manner, using the switching unit 167d to check whether the internal spaces 152 and 162d of the drug is made negative by the syringe S, it is possible to determine whether a blood vessel is punctured with the distal end of the needle member 150 included in the injection unit 140d.

Note that the invention is not limited to the embodiments, and various changes can be made within the scope of the claims. In the first embodiment, the first sensor 171 and the second sensor 172 included in the first measurement unit 170 is fixed with an adhesive or by thermal fusion or the like to the internal space 152 of the needle member 150 included in the injection unit 140.

As long as a pressure loss in the elongated internal space 152 is measured, the first sensor 171 and the second sensor 172 are not necessarily fixed to the internal space 152 of the needle member 150. The first sensor 171 and the second sensor 172 may be placed in the internal space of the injection unit such as the needle member during a surgery.

Furthermore, in the first embodiment, the injection unit 140 includes the needle member 150 and the connecting member 160. As long as a pressure loss in the elongated internal space 152 is detected, for example, an injection unit 140 according to another embodiment of the invention may include one of the needle member 150 and the needle member 150a and does not include the connecting member 160.

In addition, the auditory alert unit 182 is illustrated as a member that notifies a user when a difference of values measured by the first sensor 171 and the second sensor 172 exceeds a predetermined threshold. However, the notification is not limited to the above situation. In addition to the above situation, the auditory alert unit 182 may notify a user when a value measured by the first sensor 171 disposed in the distal end side of the internal spaces 152 and 162 becomes equal to or more than a specific level or when a measured value decreases rapidly.

Furthermore, a way to notify an operator or other users in the above cases is not limited to sounds, and characters may also be used. Moreover, a way to notify an operator or other users is not limited to images and sounds. Like a syringe pump, for example, in a case where a motor or the like is used to move the pressing member 120 relative to the barrel 110 for delivering a drug and the motor and other parts are controlled by the control unit 190, a user may be notified of the situation by, for example, the control unit 190 stopping drug delivery or adjusting the speed of drug delivery. In the first embodiment, the medical device 100 is illustrated as a member including the barrel 110, but the medical device may have a component other than the barrel 110 that is connected to the injection unit as long as it contains a drug.

This application is based on JP 2021-048594 B1 filed on March 23, 2021, the contents of which are incorporated by reference in their entirety.

### Reference Signs List

110 Barrel (drug container)
120 Pressing member
140, 140a, 140c, 140d Injection unit
150, 150a Needle member
151, 151a Outlet
160, 160c, 160d Connecting member
163 First opening (part connected to barrel)
164 Second opening (part that guides wire from first measurement unit)
170 First measurement unit
171 First sensor
172 Second sensor
180 Notification unit
181 Visual alert unit
182 Auditory alert unit
210 Second measurement unit
W Wire

## Claims

1. A medical device comprising:
an injection unit connectable to a drug container that is capable of containing a drug and including a channel that allows passage of the drug; and
a first measurement unit that measures a pressure at a first part in a distal end side of the channel and at a second part closer to a proximal end side of the channel than the first part.

2. The medical device according to claim 1,
wherein the first measurement unit is capable of measuring the pressure while being connected to a wire, and
the injection unit includes
a needle member provided with the channel and capable of puncturing a body of a patient and
a connecting member provided with the channel and connected to the needle member in a proximal end side of the needle member, the connecting member having a part connected to the drug container and a part that guides the wire from the first measurement unit being different from each other.

3. The medical device according to claim 1 or 2, wherein the injection unit has a tubular shape provided with the channel and having a closed distal end, the tubular shape having a lateral surface provided with an outlet for releasing the drug from the drug container.

4. The medical device according to any one of claims 1 to 3, further comprising a second measurement unit that measures a pressure of a fluid existing around the injection unit being in contact with the fluid, the second measurement unit being disposed outside the injection unit.

5. The medical device according to any one of claims 1 to 4, wherein the first measurement unit includes a first sensor disposed in the first part in the channel and a second sensor disposed in the second part in the channel.

6. The medical device according to any one of claims 1 to 5, wherein the first measurement unit is fixed to the channel.

7. The medical device according to claim 5,
wherein the channel is formed in an elongated shape, and
the second part provided with the second sensor is larger than the first part provided with the first sensor in cross section intersecting with a longitudinal direction.

8. The medical device according to any one of claims 1 to 7, wherein the injection unit further includes a switching unit that switches between connection with an instrument capable of making a pressure of the channel negative and disconnection from the instrument.

9. The medical device according to any one of claims 1 to 8, further comprising a visual alert unit that visually displays a measured value associated with the pressure measured by the first measurement unit.

10. The medical device according to any one of claims 1 to 9, further comprising a notification unit that notifies a user when a value measured by the first measurement unit satisfies a specific condition.
